# EUROPEAN PATENT APPLICATION

(11) **EP 4 102 259 A1**
(43) Date of publication of application: **14.12.2022**
(21) Application number: 21751313.4
(22) Date of filing: 01.02.2021
(51) Int. Cl.: G01T 7/00, H04N 5/32, H04N 5/374, A61B 6/00

(54) **RADIATION DETECTOR**

(30) Priority: 06.02.2020 JP 2020018897; 26.01.2021 JP 2021010236
(71) Applicant: Canon Electron Tubes & Devices Co., Ltd., Otawara-shi, Tochigi 324-0036 (JP)
(72) Inventor: FUJITA, Shuichi, Otawara-shi, Tochigi 324-0036 (JP)
(74) Representative: Henkel & Partner mbB
(86) International application number: PCT/JP2021/003516
(87) International publication number: WO 2021/157520

(57) **Abstract**

This radiation detector is provided with: a plurality of control lines extending in a first direction; a plurality of data lines extending in a second direction which intersects the first direction; photoelectric conversion units which are electrically connected to the corresponding control line and the corresponding data line, and each of which includes a photoelectric conversion element; scintillators provided on the plurality of photoelectric conversion units; a bias line which is electrically connected to the plurality of photoelectric conversion elements; a voltage generating circuit which is electrically connected to the bias line; and a radiation incidence determining circuit which is electrically connected to the bias line, and which detects a voltage change that occurs when incidence of radiation begins.

## Description

### [Technical Field]

Embodiments of the invention relate to a radiation detector.

### [Background Art]

An X-ray detector is an example of a radiation detector. The X-ray detector includes, for example, an array substrate that includes multiple photoelectric converters, and a scintillator that is provided on the multiple photoelectric converters and converts X-rays into fluorescence. Also, the photoelectric converter includes a photoelectric conversion element that converts the fluorescence from the scintillator into a charge, a thin film transistor that switches between storing and discharging the charge, etc.

Generally, an X-ray detector reads image data as follows. First, the incidence start of the X-rays is recognized by a signal input from the outside. Then, after a predetermined amount of time has elapsed, the thin film transistors of the photoelectric converters performing reading are set to the ON-state, and the stored charge is read as image data. However, to do so, a synchronous interface for synchronizing the X-ray detector with an external device such as an X-ray source or the like is necessary.

Here, the values of the image data obtained by the scintillator and the photoelectric conversion element are different between when the X-rays are incident and when the X-rays are not incident. Therefore, technology has been proposed in which the incidence start of the X-rays is detected by detecting the difference between the values of the image data when the X-rays are not incident and the values of the image data when the X-rays are incident. However, such technology requires an imaging preparation stage of pre-acquiring and storing image data when the X-rays are not incident as a base of comparison, and requires constantly acquiring the image data and performing a comparison calculation.

Therefore, electrical power is constantly consumed even in standby when X-rays are not incident, and the power consumption is undesirably large. In such a case, it is difficult to use a portable X-ray detector having a battery as the power supply for a long period of time because the consumption of the battery increases. Also, the temperature of the circuit easily rises due to the large power consumption, and there are cases where the use of the X-ray detector is limited in high-temperature environments. Furthermore, large-capacity image memory is necessary because it is necessary to store the comparison image.

It is therefore desirable to develop a radiation detector in which the power consumption when detecting the incidence of radiation can be suppressed.

### [Prior Art Documents]

### [Patent Literature]

[Patent Document 1]
JP-A 2019-020141 (Kokai)

### [Summary of Invention]

### [Technical Problem]

A problem to be solved by the invention is to provide a radiation detector in which the power consumption when detecting the incidence of radiation can be suppressed.

### [Solution to Problem]

A radiation detector according to an embodiment includes a plurality of control lines extending in a first direction, a plurality of data lines that extend in a second direction crossing the first direction, a photoelectric converter that includes a photoelectric conversion element and is electrically connected to a corresponding control line and a corresponding data line, a scintillator provided on a plurality of the photoelectric converters, a bias line electrically connected to a plurality of the photoelectric conversion elements, a voltage generation circuit electrically connected to the bias line, and a radiation incidence determination circuit that is electrically connected to the bias line and detects a change of a voltage occurring at an incidence start of radiation.

### [Brief Description of Drawings]

[FIG. 1]

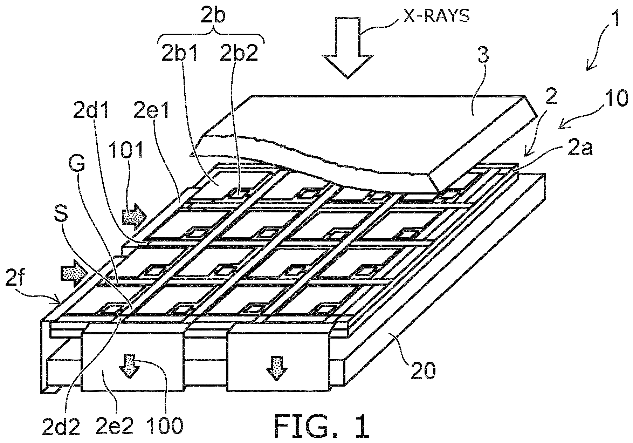

   FIG. 1 is a schematic perspective view for illustrating an X-ray detector according to the embodiment.
[FIG. 2]

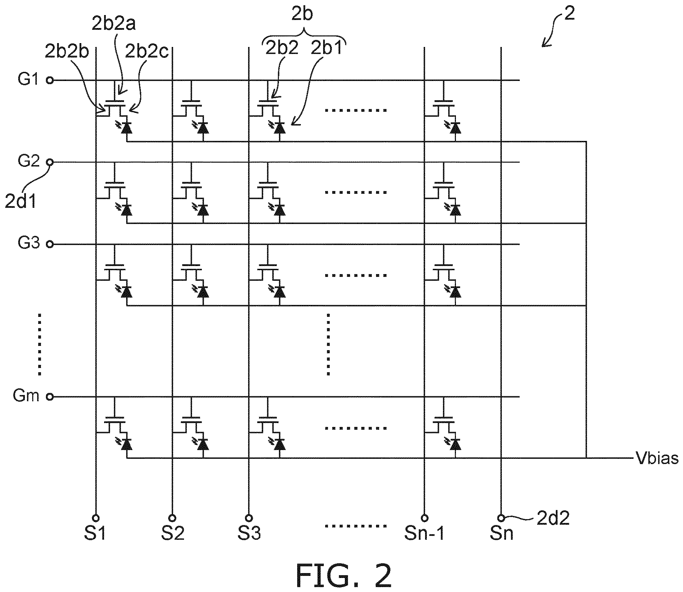

   FIG. 2 is a circuit diagram of an array substrate.
[FIG. 3]

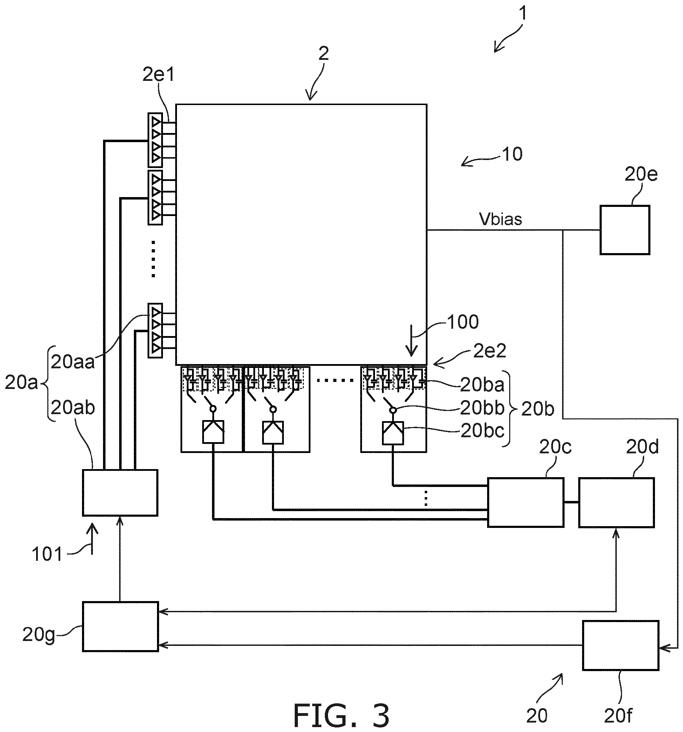

   FIG. 3 is a block diagram of the X-ray detector.
[FIG. 4]

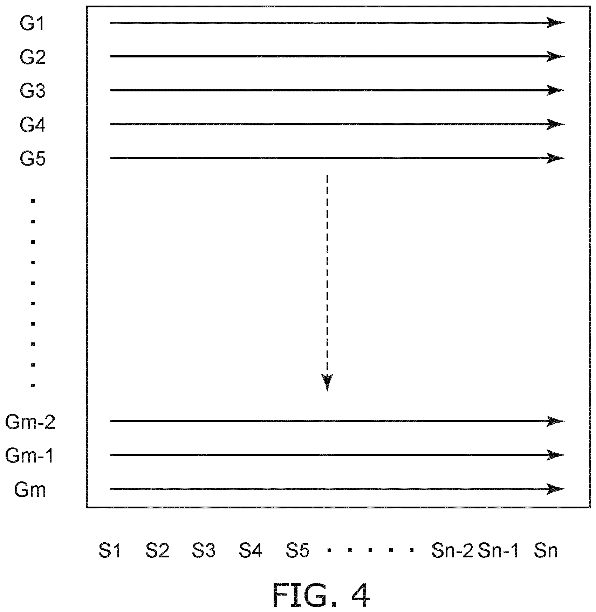

   FIG. 4 is a sequence diagram for illustrating the reading of image data.
[FIG. 5]

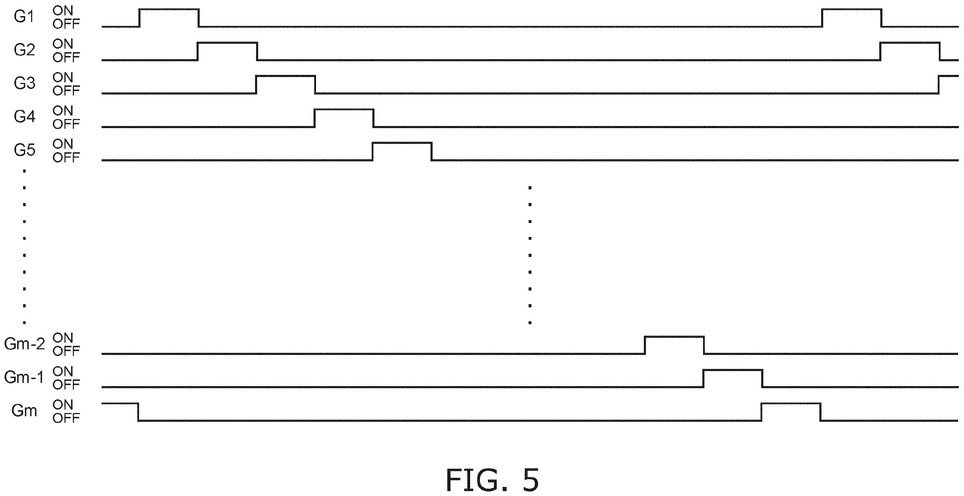

   FIG. 5 is a timing chart for illustrating the reading of the image data.
[FIG. 6]

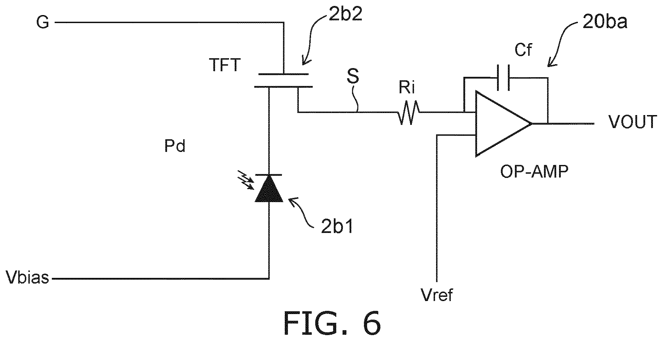

   FIG. 6 is an internal equivalent circuit of an acquisition operation of an X-ray image.
[FIG. 7]

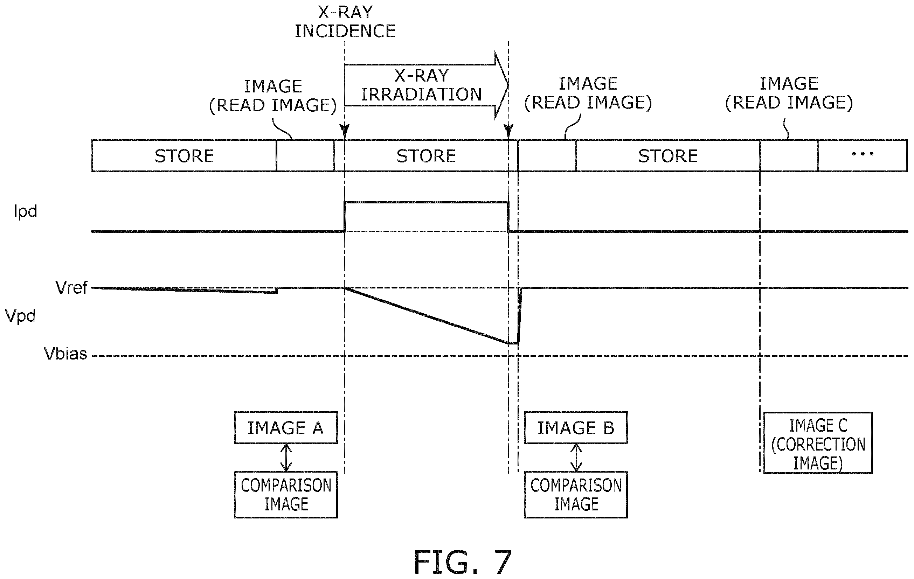

   FIG. 7 is a sequence diagram for illustrating a determination of the incidence start of the X-rays according to a comparative example.
[FIG. 8]

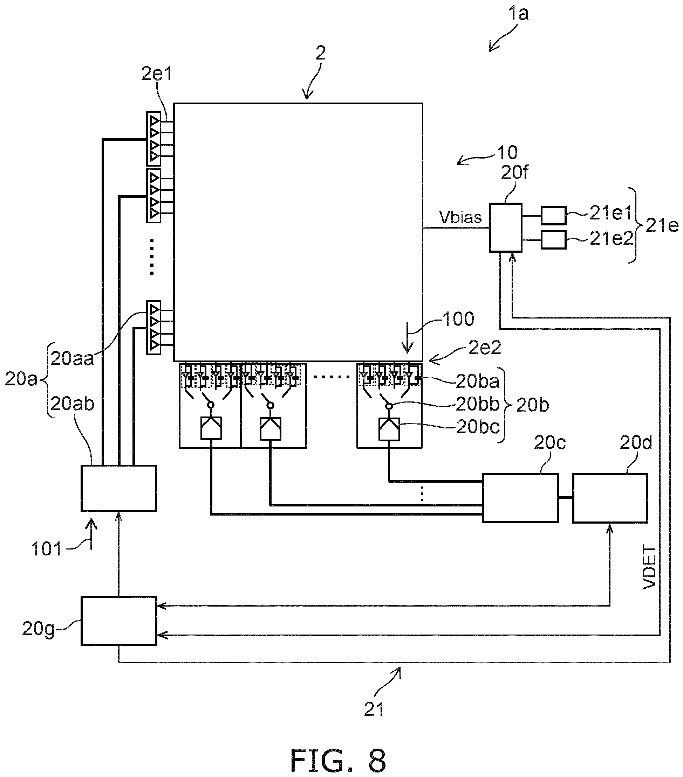

   FIG. 8 is a block diagram of an X-ray detector according to another embodiment.
[FIG. 9]

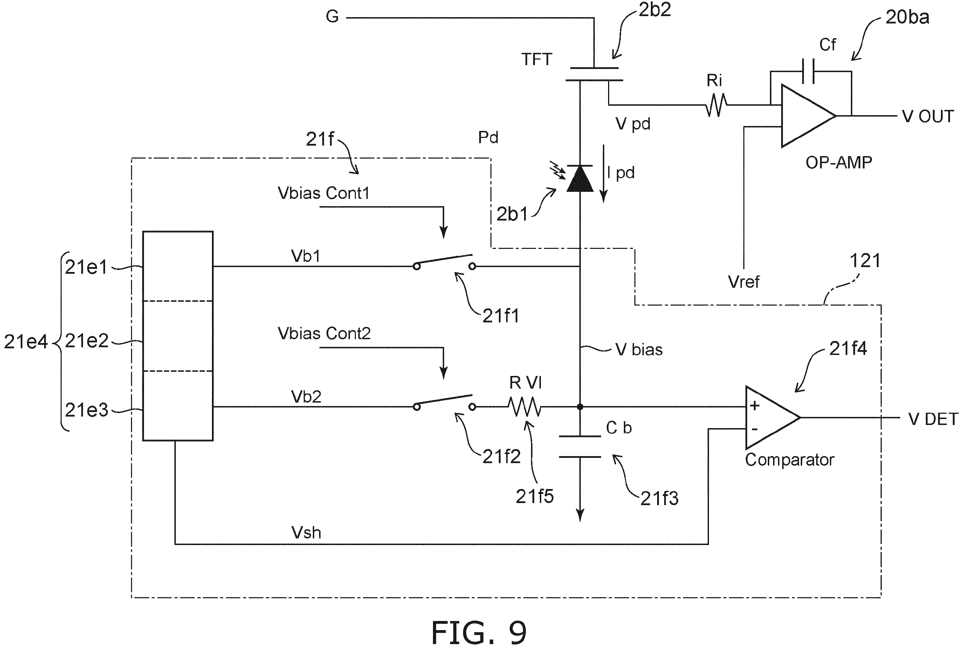

   FIG. 9 is a circuit diagram for illustrating an X-ray incidence determination circuit.
[FIG. 10]

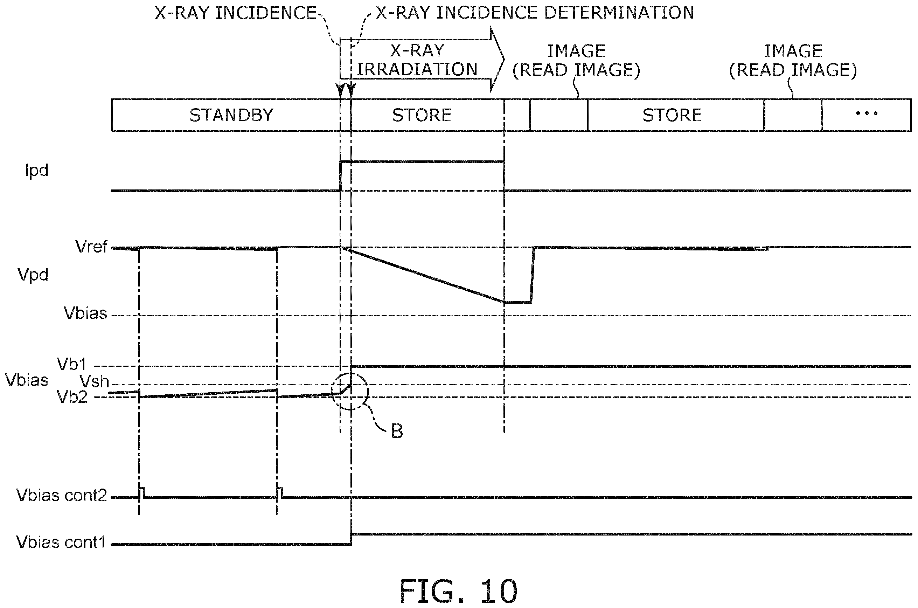

   FIG. 10 is a sequence diagram for illustrating the determination of the incidence start of the X-rays.
[FIG. 11]

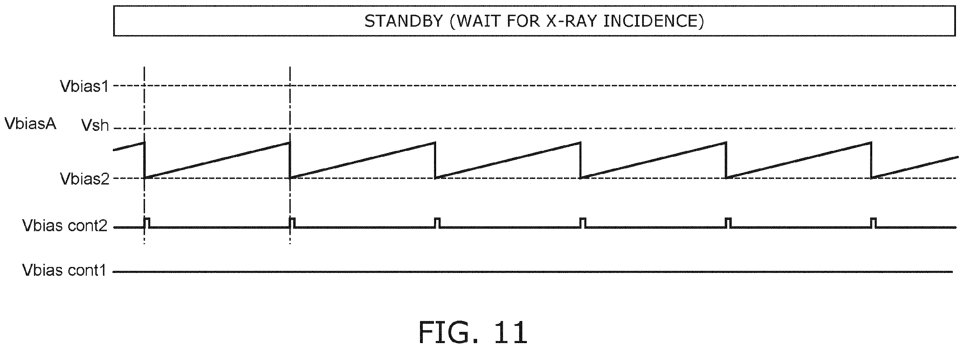

   FIG. 11 is a sequence diagram for illustrating the standby state.
[FIG. 12]

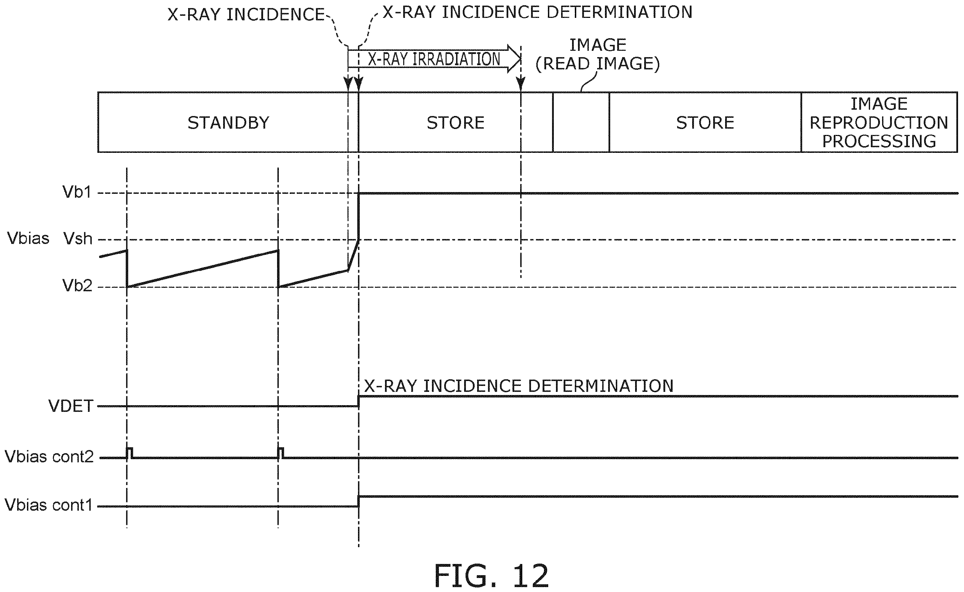

   FIG. 12 is a sequence diagram for illustrating the imaging of the X-ray image.
[FIG. 13]

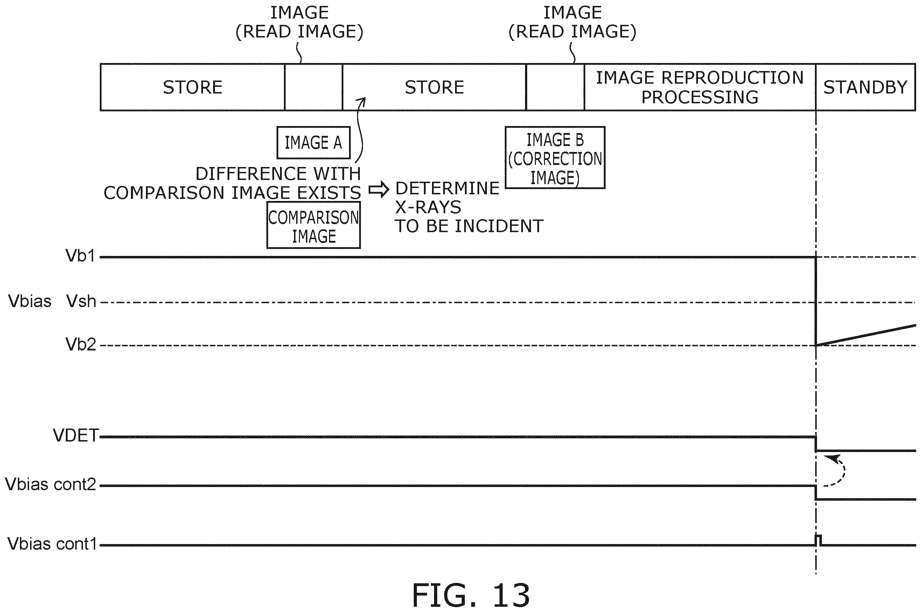

   FIG. 13 is a sequence diagram when there is a difference between the comparison image and the imaged X-ray image.
[FIG. 14]

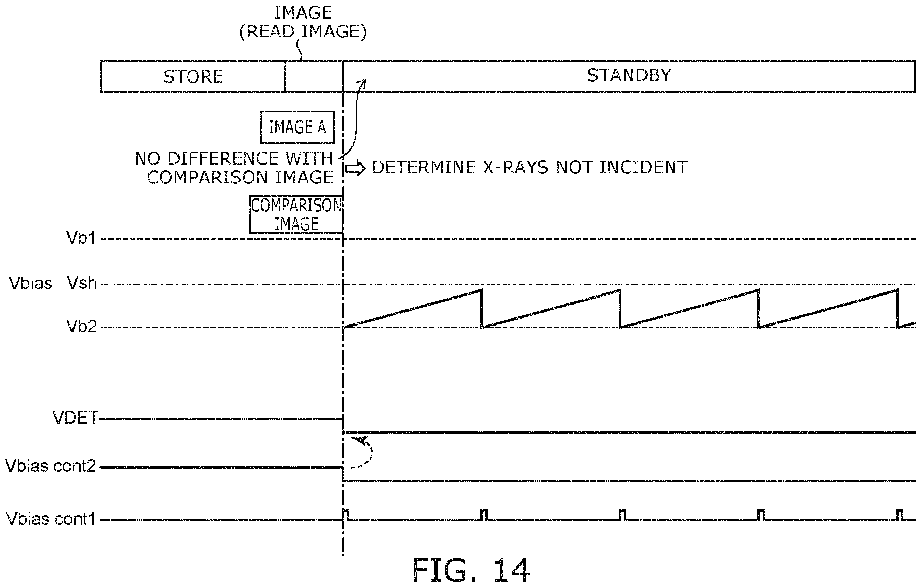

   FIG. 14 is a sequence diagram when there is no difference between the comparison image and the imaged X-ray image.

### [Description of Embodiments]

Embodiments will now be illustrated with reference to the drawings. Similar components in the drawings are marked with the same reference numerals; and a detailed description is omitted as appropriate.

A radiation detector according to the embodiment is applicable to various radiation other than X-rays such as γ-rays, etc. Herein, as an example, the case relating to X-rays is described as a typical example of radiation. Accordingly, applications to other radiation also are possible by replacing "X-ray" of embodiments described below with "other radiation".

Also, for example, an X-ray detector 1 can be used in general medical care, etc. However, the applications of the X-ray detector 1 are not limited to general medical care, etc.

FIG. 1 is a schematic perspective view for illustrating the X-ray detector 1 according to the embodiment.

FIG. 2 is a circuit diagram of an array substrate 2.

FIG. 3 is a block diagram of the X-ray detector 1.

FIG. 4 is a sequence diagram for illustrating the reading of image data 100.

FIG. 5 is a timing chart for illustrating the reading of the image data 100.

As shown in FIGS. 1 to 3, the X-ray detector 1 can include an X-ray detection module 10 and a circuit board 20. Also, the X-ray detector 1 can include a not-illustrated housing. The X-ray detection module 10 and the circuit board 20 can be provided inside the housing. For example, a support plate can be provided inside the housing; the X-ray detection module 10 can be provided at the surface of the support plate at the X-ray incident side; and the circuit board 20 can be provided at the surface of the support plate at the side opposite to the X-ray incident side.

The array substrate 2 and a scintillator 3 can be provided in the X-ray detection module 10.

The array substrate 2 can include a substrate 2a, a photoelectric converter 2b, a control line (or gate line) G, a data line (or signal line) S, an interconnect pad 2d1, an interconnect pad 2d2, and a protective layer 2f. The numbers of the photoelectric converters 2b, the control lines G, the data lines S, etc., are not limited to those illustrated.

The substrate 2a is plate-shaped and can be formed from glass such as alkali-free glass, etc. The planar shape of the substrate 2a can be quadrilateral.

Multiple photoelectric converters 2b can be provided at one surface side of the substrate 2a. The photoelectric converter 2b is rectangular and can be provided in a region defined by the control lines G and the data lines S. The multiple photoelectric converters 2b can be arranged in a matrix configuration. For example, one photoelectric converter 2b corresponds to one pixel (pixel) of the X-ray image.

Each of the multiple photoelectric converters 2b can include a photoelectric conversion element 2b1, and a thin film transistor (TFT; Thin Film Transistor) 2b2 that is a switching element. Also, a storage capacitor that stores the converted signal charge can be included in the photoelectric conversion element 2b1. However, according to the capacitance of the photoelectric conversion element 2b1, the photoelectric conversion element 2b1 also can be used as the storage capacitor. A case will now be illustrated in which the photoelectric conversion element 2b1 is used as the storage capacitor.

The photoelectric conversion element 2b1 can be, for example, a photodiode, etc.

The thin film transistor 2b2 can switch between storing and discharging charge to and from the photoelectric conversion element 2b1 that functions as the storage capacitor. The thin film transistor 2b2 can include a gate electrode 2b2a, a drain electrode 2b2b, and a source electrode 2b2c. The gate electrode 2b2a of the thin film transistor 2b2 can be electrically connected with the corresponding control line G. The drain electrode 2b2b of the thin film transistor 2b2 can be electrically connected with the corresponding data line S. The source electrode 2b2c of the thin film transistor 2b2 can be electrically connected to the corresponding photoelectric conversion element 2b1. Also, the anode side of the photoelectric conversion element 2b1 can be electrically connected to a bias line Vbias.

Multiple control lines G can be arranged parallel to each other at a prescribed spacing. For example, the multiple control lines G extend in a row direction (corresponding to an example of a first direction) and are arranged in a column direction (corresponding to an example of a second direction) crossing the row direction. One control line G can be electrically connected with one of the multiple interconnect pads 2d1 provided at the peripheral edge vicinity of the substrate 2a. One of the multiple interconnects provided in a flexible printed circuit board 2e1 can be electrically connected to one interconnect pad 2d1. The other ends of the multiple interconnects provided in the flexible printed circuit board 2e1 each can be electrically connected with a gate drive circuit 20a provided in the circuit board 20.

Multiple data lines S can be arranged parallel to each other at a prescribed spacing. For example, the data lines S extend in the column direction and are arranged in the row direction. One data line S can be electrically connected with one of the multiple interconnect pads 2d2 provided at the peripheral edge vicinity of the substrate 2a. One of the multiple interconnects provided in a flexible printed circuit board 2e2 can be electrically connected to one interconnect pad 2d2. The other ends of the multiple interconnects provided in the flexible printed circuit board 2e2 each can be electrically connected with a signal detection circuit 20b provided in the circuit board 20.

For example, the control line G, the data line S, and the bias line Vbias can be formed using a low-resistance metal such as aluminum, chrome, etc.

The protective layer 2f can cover the photoelectric converter 2b, the control line G, the data line S, and the bias line Vbias. The protective layer 2f can be formed from an insulating material.

The scintillator 3 can be provided on the multiple photoelectric converters 2b. The scintillator 3 can convert the incident X-rays into fluorescence. The scintillator 3 can be provided to cover the region (the effective pixel region) in which the multiple photoelectric converters 2b are provided. For example, the scintillator 3 can be formed using cesium iodide (CsI):thallium (TI), sodium iodide (NaI):thallium (TI), cesium bromide (CsBr):europium (Eu), etc. The scintillator 3 can be formed using vacuum vapor deposition. By forming the scintillator 3 by using vacuum vapor deposition, the scintillator 3 that is made of an aggregate of multiple columnar crystals is formed.

Also, for example, the scintillator 3 can be formed using terbium-activated sulfated gadolinium (Gd₂O₂S/Tb or GOS), etc. In such a case, a trench portion having a matrix configuration can be provided so that a quadrilateral prism-shaped scintillator 3 is provided for each of the multiple photoelectric converters 2b.

Also, a reflective layer can be provided at the X-ray incident side of the scintillator 3. The reflective layer reflects the light of the fluorescence generated by the scintillator 3 that travels toward the side opposite to the side at which the photoelectric converters 2b are provided, and causes the light to travel toward the photoelectric converters 2b.

A moisture-resistant part that covers the scintillator 3 and the reflective layer also can be provided.

The circuit board 20 can be provided at the side opposite to the side at which the scintillator 3 of the array substrate 2 is provided. The circuit board 20 can be electrically connected with the X-ray detection module 10 (the array substrate 2).

As shown in FIG. 3, the circuit board 20 can include the gate drive circuit 20a, the signal detection circuit 20b, memory 20c, an image configuration circuit 20d, a voltage generation circuit 20e, an X-ray incidence determination circuit 20f, and a controller 20g. These components can be provided in one substrate or can be provided separately in multiple substrates.

The gate drive circuit 20a can switch between an ON-state and an OFF-state of the thin film transistors 2b2. The gate drive circuit 20a can include a row selection circuit 20ab and multiple gate drivers 20aa.

A control signal 101 can be input from the controller 20g to the row selection circuit 20ab. The row selection circuit 20ab can input the control signal 101 to the corresponding gate driver 20aa according to the scan direction of the X-ray image.

The gate driver 20aa can input the control signal 101 to the corresponding control line G.

For example, as shown in FIGS. 4 and 5, the gate drive circuit 20a can sequentially input the control signal 101 to control lines G1 to Gm via the flexible printed circuit board 2e1. The thin film transistors 2b2 are set to the ON-state by the control signals 101 input to the control lines G, and the charge (the image data 100) can be read from the photoelectric conversion elements 2b1 that function as the storage capacitors.

The signal detection circuit 20b can read the image data 100 from the photoelectric converters 2b when the thin film transistors 2b2 are in the ON-state. The signal detection circuit 20b can include multiple integrating amplifiers 20ba, multiple selection circuits 20bb, and multiple AD converters 20bc.

One integrating amplifier 20ba can be electrically connected with one data line S. The integrating amplifiers 20ba can sequentially receive the image data 100 from the photoelectric converters 2b. Then, the integrating amplifier 20ba can integrate the current flowing in a constant amount of time and can output a voltage corresponding to the integral to the selection circuit 20bb. Thus, the value (the charge amount) of the current flowing through the data line S within a prescribed interval can be converted into a voltage value. In other words, the integrating amplifier 20ba can convert image data information corresponding to the intensity distribution of the fluorescence generated by the scintillator 3 into potential information.

The selection circuit 20bb can sequentially read the image data 100 converted into the potential information by selecting the integrating amplifier 20ba that performs the reading.

The AD converter 20bc can sequentially convert the read image data 100 into a digital signal. The image data 100 that is converted into the digital signal can be stored in the memory 20c.

For example, the signal detection circuit 20b can sequentially read the image data 100 for each of data lines S1 to Sn via the flexible printed circuit board 2e2.

An internal equivalent circuit of such an acquisition operation of the X-ray image is as illustrated in FIG. 6.

For example, the memory 20c can store a control program that controls the circuits provided in the circuit board 20. For example, the memory 20c also can store data such as thresholds necessary when executing the control program, etc. The memory 20c also can temporarily store the image data 100 that is converted into the digital signals.

The image configuration circuit 20d can configure an X-ray image based on the image data 100 stored in the memory 20c. The image configuration circuit 20d also can be provided outside the X-ray detector 1. When the image configuration circuit 20d is provided outside the X-ray detector 1, the data communication between the circuit board 20 and the image configuration circuit 20d can be performed wirelessly and can be performed via an interconnect, etc. The image configuration circuit 20d can transmit the data of the configured X-ray image to a display device and/or another device provided outside the X-ray detector 1.

As shown in FIG. 2, the voltage generation circuit 20e can be electrically connected to the bias line Vbias. For example, the voltage generation circuit 20e generates a bias voltage. The voltage generation circuit 20e stores a prescribed charge in the multiple photoelectric conversion elements 2b1 that function as the storage capacitors. The voltage generation circuit 20e can be, for example, a DC power supply, etc. When X-rays are incident on the X-ray detector 1, fluorescence is generated by the scintillator 3, and the generated fluorescence is incident on the photoelectric conversion elements 2b1. When the fluorescence is incident on the photoelectric conversion elements 2b1, a charge (electrons and holes) is generated by the photoelectric effect; and the stored charge (the heterogeneous charge) is reduced by the combination of the generated charge and the stored charge. The charge after the reduction can be read as the image data 100.

Here, when the X-rays are incident on the X-ray detector 1, the charge that is stored in the photoelectric conversion elements 2b1 decreases; therefore, the incidence start of the X-rays can be detected by detecting the charge.

The X-ray incidence determination circuit 20f can be electrically connected to the bias line Vbias. The X-ray incidence determination circuit 20f can determine the incidence start of the X-rays by reading the charge from the photoelectric conversion elements 2b1. The X-ray incidence determination circuit 20f can detect the change of the voltage occurring at the incidence start of the X-rays. The X-ray incidence determination circuit 20f can determine that the incidence of the X-rays has started if the detected voltage value is less than a threshold, and can determine that the incidence of the X-rays has not started when the detected voltage value is greater than the threshold. When the incidence of the X-rays is determined to have started, the X-ray incidence determination circuit 20f can transmit a signal to the controller 20g indicating that the incidence of the X-rays has started.

The controller 20g can control each circuit provided in the circuit board 20 based on the control program stored in the memory 20c. The controller 20g can include, for example, an arithmetic element such as a CPU (Central Processing Unit), etc.

The determination of the incidence start of the X-rays will now be described further.
FIG. 7 is a sequence diagram for illustrating a determination of the incidence start of the X-rays according to a comparative example.

As shown in FIG. 7, a current Ipd is generated in the photoelectric conversion element 2b1 when the X-rays are incident on the X-ray detector. Also, a voltage Vpd of the photoelectric conversion element 2b1 gradually decreases because the charge stored in the photoelectric conversion element 2b1 gradually decreases. Therefore, the incidence start of the X-rays can be detected by reading the charge stored in the photoelectric conversion elements 2b1 as the image data 100 and determining the difference with a "comparison image".

For example, there is no change of the stored charge before the incidence of the X-rays; therefore, the difference between the values of the image data 100 of the "image A" that is read and the values of the image data 100 of the predetermined "comparison image" is small. In contrast, a change of the stored charge occurs after the incidence of the X-rays; therefore, the difference between the values of the image data 100 of the "image B" that is read and the values of the image data 100 of the "comparison image" is large. Therefore, the incidence start of the X-rays can be detected based on the difference of the values of the image data 100.

However, because it is difficult to predict the incidence timing of the X-rays, it is necessary to continuously acquire and compare the image data 100 of the X-ray image to be determined. It is therefore necessary to constantly operate the gate drive circuit 20a and the signal detection circuit 20b. As a result, power consumption is large even in standby when X-rays are not incident. Also, there are cases where the use of the X-ray detector 1 in high-temperature environments is limited due to the temperature rise due to the heat generation. Furthermore, a large-capacity image memory is necessary because it is necessary to store the data of one "comparison image".

In the X-ray detector 1 according to the embodiment, the X-ray incidence determination circuit 20f determines the incidence start of the X-rays by detecting the change of the voltage occurring in the bias line Vbias. It is therefore unnecessary to constantly operate the gate drive circuit 20a and the signal detection circuit 20b that have high power consumption amounts; therefore, the power consumption when detecting the incidence of the X-rays can be suppressed. Also, because the rise of the temperature of the circuit can be suppressed, the limit of the use of the X-ray detector in high-temperature environments can be suppressed. Also, it is unnecessary to provide image memory that stores the data of the "comparison image".

After the incidence of the X-rays is detected, it is sufficient for the voltage generation circuit 20e to re-store the charge in the photoelectric conversion elements 2b1 functioning as the storage capacitors. The reading of the image data 100 becomes possible by the re-stored charge being reduced by the charge generated by the photoelectric conversion elements 2b1.

FIG. 8 is a block diagram of an X-ray detector 1a according to another embodiment.

FIG. 9 is a circuit diagram for illustrating an X-ray incidence determination circuit 21f.

As shown in FIG. 8, the X-ray detector 1a can include the X-ray detection module 10 and a circuit board 21. The circuit board 21 can include the gate drive circuit 20a, the signal detection circuit 20b, the memory 20c, the image configuration circuit 20d, a voltage generation circuit 21e, the X-ray incidence determination circuit 21f, and the controller 20g.

As shown in FIG. 8, the voltage generation circuit 21e can include a circuit 21e1 that generates a first bias voltage Vb1, and a circuit 21e2 that generates a second bias voltage Vb2.

It is important for the circuit 21e2 generating the second bias voltage Vb2 to be set to an extremely large value so that the impedance of the supply power line is at the level of several tens of kQ.

The circuit 21e1 that generates the first bias voltage Vb1 can be electrically connected to the bias line Vbias via a switch 21f1 of the X-ray incidence determination circuit 21f. The circuit 21e1 that generates the first bias voltage Vb1 applies the first bias voltage Vb1 to the bias line Vbias. The first bias voltage Vb1 can be a bias voltage used when imaging the X-ray image. For example, the circuit 21e1 that generates the first bias voltage Vb1 may be the voltage generation circuit 20e described above.

The circuit 21e2 that generates the second bias voltage Vb2 can be electrically connected to the bias line Vbias via a switch 21f2 of the X-ray incidence determination circuit 21f. The circuit 21e2 that generates the second bias voltage Vb2 applies, to the bias line Vbias, the second bias voltage Vb2 that is different from the first bias voltage Vb1. The second bias voltage Vb2 can be a bias voltage used when detecting the incidence start of the X-rays. For example, the potential of the circuit 21e2 generating the second bias voltage Vb2 can be less than the potential of the circuit 21e1 generating the first bias voltage Vb1 (the second bias voltage Vb2 can be less than the first bias voltage Vb1). For example, although the setting is dependent on the impedance inside the power supply, the potential of the circuit 21e2 generating the second bias voltage Vb2 is set to be about 1.5 times less than the potential of the circuit 21e1 generating the first bias voltage Vb1.

In such a case, the circuit 21e1 that generates the first bias voltage Vb1 and the circuit 21e2 that generates the second bias voltage Vb2 may be integrated. For example, the first bias voltage Vb1 from the circuit 21e1 generating the first bias voltage Vb1 may be used as the second bias voltage Vb2 by using a resistance or the like. In such a case, the resistance or the like is the circuit 21e2 generating the second bias voltage Vb2.

Also, as shown in FIG. 9, a voltage generation circuit 21e4 that includes the circuit 21e1 generating the first bias voltage Vb1, the circuit 21e2 generating the second bias voltage Vb2, and a circuit 21e3 generating a threshold voltage Vsh applied to a comparator 21f4 can be provided.

The circuit 21e1 that generates the first bias voltage Vb1, the circuit 21e2 that generates the second bias voltage Vb2, and the circuit 21e3 that generates the threshold voltage Vsh may be individually provided or may be, for example, integrated as an integrated circuit. Details of the integration in an integrated circuit are described below.

The X-ray incidence determination circuit 21f can include the switch 21f1, the switch 21f2, a capacitor 21f3, and the comparator 21f4.

The switch 21f1 and the switch 21f2 can perform ON/OFF operations based on signals from the controller 20g. In other words, the controller 20g can control the switches 21f1 and 21f2. Therefore, the circuit 21e1 that generates the first bias voltage Vb1 and the circuit 21e2 that generates the second bias voltage Vb2 can store charge in the capacitor 21f3 and the photoelectric conversion element 2b1 functioning as the storage capacitor at the determined timing.

The capacitor 21f3 can be electrically connected to the bias line Vbias. The capacitor 21f3 can be provided to capture micro current changes as the voltage when detecting the incidence start of the X-rays. The capacitor 21f3 can maintain the first bias voltage Vb1 or the second bias voltage Vb2 for a short period of time. Although the capacitor 21f3 can be omitted according to the capacitance of the photoelectric conversion element 2b1, it is easy to detect the incidence start of the X-rays with high accuracy when the capacitor 21f3 is provided.

The second bias voltage Vb2 is applied to the capacitor 21f3 (the comparator 21f4) via a resistance 21f5. The comparator 21f4 can be electrically connected to the capacitor 21f3. The comparator 21f4 can compare the threshold voltage Vsh and the voltage of the capacitor 21f3.

Here, for example, when the circuit 21e1 that generates the first bias voltage Vb1, the circuit 21e2 that generates the second bias voltage Vb2, and the circuit 21e3 that generates the threshold voltage Vsh described above are configured by combining elements such as discrete semiconductor devices or the like on a substrate, there are cases where voltage fluctuation occurs due to temperature fluctuation, fluctuation of the characteristics of the individual components, etc. Also, a wiring pattern for electrically connecting the individual components is necessary, and the wiring pattern has an impedance; therefore, there is a risk that the induction noise may increase according to the other circuits and/or the surrounding environment.

It is therefore favorable for at least the circuit 21e1 generating the first bias voltage Vb1 and the circuit 21e2 generating the second bias voltage Vb2 to be integrated as an integrated circuit.

Also, it is more favorable for the circuit 21e1 generating the first bias voltage Vb1, the circuit 21e2 generating the second bias voltage Vb2, and the circuit 21e3 generating the threshold voltage Vsh to be integrated as an integrated circuit. For example, as shown in FIG. 9, by integrating the circuit 21e1 generating the first bias voltage Vb1, the circuit 21e2 generating the second bias voltage Vb2, and the circuit 21e3 generating the threshold voltage Vsh as an integrated circuit 121, the fluctuation of the characteristics of the individual components can be suppressed, and the interconnects also can be completed inside the integrated circuit 121. Therefore, the voltage fluctuation and the induction noise can be suppressed. Also, parameters such as the resistance constant, etc., can be trimmed with high accuracy. Also, by using the integrated circuit 121, each circuit is provided inside the same package; therefore, the temperature fluctuation can be prevented from being different between the circuits. Therefore, the characteristics can be uniform.

Also, as shown in FIG. 9, the second bias voltage Vb2 is applied to the comparator 21f4 via the resistance 21f5. Because the resistance 21f5 is connected in series to the comparator 21f4, a voltage that is proportional to the current that flows is generated. Because the determination of the incidence start of the X-rays uses the voltage of the capacitor 21f3 electrically connected to the resistance 21f5, if a resistance value R_VI of the resistance 21f5 fluctuates, there is a risk that the voltage of the capacitor 21f3 may fluctuate and the determination accuracy of the incidence start of the X-rays may decrease.

The characteristics of the comparator 21f4 also change when a difference of the input current occurs.

Therefore, the determination accuracy of the incidence start of the X-rays can be increased by setting the resistance value of the resistance 21f5, the input current value of the comparator 21f4, etc., to be substantially constant.

In such a case, if the circuit that generates the first bias voltage Vb1, the circuit that generates the second bias voltage Vb2, and the circuit 21e3 that generates the threshold voltage Vsh are in the integrated circuit 121, the resistance 21f5 and the comparator 21f4 also are provided in the integrated circuit 121. It is possible to further increase the determination accuracy of the incidence start of the X-rays by trimming the resistance 21f5 when providing in the integrated circuit 121. Also, it is possible to further increase the determination accuracy of the incidence start of the X-rays because the characteristics of the comparator 21f4 can be precisely controlled when providing the comparator 21f4 in the integrated circuit 121.

FIG. 10 is a sequence diagram for illustrating the determination of the incidence start of the X-rays.

FIG. 11 is a sequence diagram for illustrating the standby state.

First, a bias voltage Vbias of the photoelectric conversion element 2b1 functioning as the storage capacitor is set to the second bias voltage Vb2 used when detecting the incidence of the X-rays. For example, the circuit 21e2 that generates the second bias voltage Vb2 is electrically connected to the bias line Vbias by setting the switch 21f2 to the ON-state. Also, the circuit 21e1 that generates the first bias voltage is blocked from the bias line Vbias by setting the switch 21f1 to the OFF-state. The circuit 21e2 that generates the second bias voltage Vb2 can charge the capacitor 21f3. When the charging of the capacitor 21f3 is completed, the switch 21f2 is set to the OFF-state. Therefore, the switch 21f2 is in the ON-state for a short period of time.

Because a leakage current Ir due to the photoelectric conversion element 2b1 itself and/or the thin film transistor 2b2 flows in the photoelectric conversion element 2b1, the charge that is stored in the capacitor 21f3 is gradually discharged, and the potential rises. Therefore, in standby (while waiting for the incidence of the X-rays), the controller 20g sets the switch 21f1 to the OFF-state and periodically repeats the ON-state and the OFF-state of the switch 21f2 as shown in FIG. 11. The recharge of the capacitor 21f3 is repeatedly performed by periodically repeating the ON-state and the OFF-state of the switch 21f2. In such a case, the period of setting the switch 21f2 to the ON-state can be set so that the voltage rise due to the leakage current Ir in standby (when a photocurrent Ix does not flow) does not cause the voltage of the capacitor 21f3 to exceed the threshold voltage Vsh for comparison. The leakage current Ir is different according to the photoelectric conversion element 2b1 and/or the thin film transistor 2b2. Therefore, the period of setting the switch 21f2 to the ON-state can be changed according to the value of the leakage current Ir. Thus, it is possible to adapt to different values of the leakage current Ir for each array substrate 2.

As shown in FIG. 10, the photocurrent Ix flows in the photoelectric conversion element 2b1 when the X-rays are incident in the standby state; therefore, the capacitor 21f3 is rapidly discharged. Therefore, the potential abruptly rises and the voltage of the capacitor 21f3 exceeds the threshold voltage Vsh for comparison; therefore, an output V_DET of the comparator 21f4 becomes ON. As a result, the incidence start of the X-rays can be detected. Although the threshold voltage Vsh is determined by the ratio of the voltage rise due to the photocurrent and the voltage rise due to the leakage current Ir flowing in the thin film transistor 2b2, in the example shown in FIG. 10, the potential of the circuit 21e2 generating the second bias voltage Vb2 is set to be 1.5 times less than the potential of the circuit 21e1 generating the first bias voltage; and by setting the threshold voltage Vsh to be a potential difference of 2 times compared to the voltage rise value due to the leakage current Ir as shown in "portion B" of FIG. 10, the detection does not occur at the leakage current Ir; and the incidence start of the X-rays can be detected with high accuracy by the abrupt change of the voltage due to the photocurrent.

As described above, it is unnecessary to operate the signal detection circuit 20b (the AD converter 20bc) and the image configuration circuit 20d in standby; therefore, the power supplies of these circuits can be set to the OFF-state. Therefore, the power consumption when detecting the incidence of the X-rays can be suppressed.

FIG. 12 is a sequence diagram for illustrating the imaging of the X-ray image.

When the incidence of the X-rays is detected as shown in FIG. 12, the bias voltage Vbias of the photoelectric conversion element 2b1 functioning as the storage capacitor switches to the first bias voltage Vb1 used when imaging the X-ray image. For example, the circuit 21e1 that generates the first bias voltage is electrically connected to the bias line Vbias by setting the switch 21f1 to the ON-state. Also, the circuit 21e2 that generates the second bias voltage Vb2 is blocked from the bias line Vbias by setting the switch 21f2 to the OFF-state.

Subsequently, the acquisition of the image data 100 and the configuration of the X-ray image can be performed similarly to the case of the X-ray detector 1 described above.

Here, there is a possibility that misdetection may occur due to noise because the voltage of the capacitor 21f3 is extremely small.

Therefore, the X-ray incidence determination circuit 21f can further compare the comparison image and the configured X-ray image. For example, the comparison image can be the image when the X-rays are not incident. For example, the comparison image can be stored in the memory 20c.

FIG. 13 is a sequence diagram when there is a difference between the comparison image and the imaged X-ray image.

It can be confirmed that the X-rays are incident when there is a difference between the comparison image and the imaged X-ray image. In such a case, as shown in FIG. 13, the imaging operation of the X-ray image continues with the first bias voltage Vb1 that is used when imaging the X-ray image as-is. For example, the X-ray incidence determination circuit 21f further compares the comparison image and the imaged X-ray image and sets the switch 21f1 to the ON-state and the switch 21f2 to the OFF-state if the prescribed difference exists between the X-ray image and the comparison image.

FIG. 14 is a sequence diagram when there is no difference between the comparison image and the imaged X-ray image.

If there is no difference between the comparison image and the imaged X-ray image, it can be determined that X-rays are not incident and a misdetection occurred. In such a case, as shown in FIG. 14, the detection operation described above can be performed by switching to the second bias voltage Vb2 used when detecting the incidence of the X-rays. In other words, when a misdetection is determined, the state can be quickly returned to the standby state.

For example, the X-ray incidence determination circuit 21f further compares the comparison image and the imaged X-ray image, and sets the switch 21f1 to the OFF-state and periodically repeats the ON-state and the OFF-state of the switch 21f2 if the prescribed difference does not exist between the X-ray image and the comparison image.

While certain embodiments of the invention have been illustrated, these embodiments have been presented by way of example only, and are not intended to limit the scope of the inventions. These novel embodiments may be embodied in a variety of other forms; and various omissions, substitutions, modifications, etc., can be made without departing from the spirit of the inventions. These embodiments and their modifications are within the scope and spirit of the inventions and are within the scope of the inventions described in the claims and their equivalents. Also, embodiments described above can be implemented in combination with each other.

### [Reference Numeral List]

1 X-ray detector
1a X-ray detector
2 array substrate
2b photoelectric converter
2b1 photoelectric conversion element
2b2 thin film transistor
3 scintillator
10 X-ray detection module
20 circuit board
20a gate drive circuit
20b signal detection circuit
20c memory
20d image configuration circuit
20e voltage generation circuit
20f X-ray incidence determination circuit
20g controller
21 circuit board
21e1 circuit generating first bias voltage
21e2 circuit generating second bias voltage
21e3 circuit generating threshold voltage
21e4 voltage generation circuit
21f X-ray incidence determination circuit
21f1 switch
21f2 switch
21f3 capacitor
21f4 comparator
100 image data

## Claims

1. A radiation detector, comprising:
a plurality of control lines extending in a first direction;
a plurality of data lines extending in a second direction, the second direction crossing the first direction;
a photoelectric converter electrically connected to a corresponding control line of the plurality of control lines and a corresponding data line of the plurality of data lines, the photoelectric converter including a photoelectric conversion element;
a scintillator provided on a plurality of the photoelectric converters;
a bias line electrically connected to a plurality of the photoelectric conversion elements;
a voltage generation circuit electrically connected to the bias line; and
a radiation incidence determination circuit electrically connected to the bias line,
the radiation incidence determination circuit detecting a change of a voltage occurring at an incidence start of radiation.

2. The radiation detector according to claim 1, wherein
the voltage generation circuit includes:
a circuit generating a first bias voltage; and
a circuit generating a second bias voltage,
the second bias voltage is less than the first bias voltage, and
the radiation incidence determination circuit includes:
a first switch electrically connected to the circuit generating the first bias voltage;
a second switch electrically connected to the circuit generating the second bias voltage;
a capacitor electrically connected to the bias line; and
a comparator electrically connected to the capacitor.

3. The radiation detector according to claim 2, wherein
the circuit generating the first bias voltage and the circuit generating the second bias voltage are integrated as an integrated circuit.

4. The radiation detector according to claim 3, wherein
the voltage generation circuit further includes a circuit generating a threshold voltage applied to the comparator, and
the circuit generating the first bias voltage, the circuit generating the second bias voltage, and the circuit generating the threshold voltage are integrated as an integrated circuit.

5. The radiation detector according to any one of claims 2 to 4, further comprising:
a controller controlling the first and second switches,
when detecting the incidence start of the radiation, the controller sets the first switch to an OFF-state and periodically repeats an ON-state and an OFF-state of the second switch, and the comparator compares a threshold voltage and a voltage of the capacitor.

6. The radiation detector according to claim 5, wherein
the radiation incidence determination circuit further compares a comparison image and a radiation image, the radiation image being imaged, and
the controller sets the first switch to the OFF-state and periodically repeats the ON-state and the OFF-state of the second switch if a prescribed difference does not exist between the radiation image and the comparison image.

7. The radiation detector according to claim 5, wherein
the radiation incidence determination circuit further compares a comparison image and a radiation image, the radiation image being imaged, and
the controller sets the first switch to an ON-state and sets the second switch to the OFF-state if a prescribed difference exists between the radiation image and the comparison image.
